# FASCICULE DE BREVET EUROPEEN

(11) **EP 2 928 452 B1**
(45) Date de publication et mention de la délivrance du brevet: **21.11.2018**
(21) Numéro de dépôt: 13818271.2
(22) Date de dépôt: 09.12.2013
(51) Int. Cl.: A61K 9/00, A61K 9/14, A61K 31/4985

(54) **COMPOSITION ORALE ET/OU BUCCALE SOUS FORME DE FILM FIN D'UN PRINCIPE ACTIF FAIBLEMENT SOLUBLE, SON PROCEDE DE PREPARATION ET SON UTILISATION**
ORALE UND/ODER BUKKALE ZUSAMMENSETZUNG IN FORM EINER DÜNNEN SCHICHT EINES SCHWACH LÖSLICHEN WIRKSTOFFES UND VERFAHREN ZUR HERSTELLUNG UND VERWENDUNG DAVON
ORAL AND/OR BUCCAL COMPOSITION IN THE FORM OF A THIN FILM OF A WEAKLY SOLUBLE ACTIVE INGREDIENT, METHOD OF PREPARING SAME AND USE OF SAME

(30) Priorité: 10.12.2012 FR 1261843
(43) Date de publication de la demande: 14.10.2015
(73) Titulaire: ETHYPHARM, 92213 Saint-Cloud Cedex (FR)
(72) Inventeur: HERRY, Catherine, F-27670 Saint-Ouen Du Tilleul (FR); BILLOET, Vincent, F-76300 Sotteville Les Rouen (FR); OURY, Pascal, 78150 Le Chesnay (FR)
(74) Mandataire: Cabinet Plasseraud
(86) Numéro de dépôt international: PCT/FR2013/053002
(87) Numéro de publication internationale: WO 2014/091134

(56) Documents cités:
- WO-A1-2010/146407
- WO-A2-2005/000265
- WO-A2-2005/013937
- WO-A2-2007/033239

## Description

La présente invention a pour objet une composition pharmaceutique orale et/ou buccale ainsi que son procédé de fabrication. De préférence cette composition pharmaceutique est présentée sous forme de film fin à usage oral se désintégrant dans la cavité buccale et est utilisée comme médicament.

L'invention comprend de manière préférée une formulation pharmaceutique orale et/ou buccale sous forme de film à désagrégation rapide permettant l'absorption d'un ou plusieurs principes actifs dont au moins l'un d'eux n'est pas ou est peu soluble dans les fluides du tractus gastro-intestinal, au moins l'un deux étant sous une forme particulaire nanonisée. Le produit fini décrit dans la présente invention est un film à désintégration orale et/ou buccale incluant une dose d'un ou plusieurs principes actifs thérapeutiques dont au moins l'un d'eux a été préalablement nanonisé.

Généralement, les films fins destinés à la voie orale et/ou buccale commercialisés sont produits par coulage et sont ensuite découpés. Une méthode alternative de fabrication est un procédé par extrusion à chaud.

Les principes actifs peu solubles appartiennent aux classes II et IV de la classification BCS (Biopharmaceutics Classification System) selon la FDA, la classe II désignant des composés ayant une grande perméabilité et une faible solubilité et la classe IV désignant des composés ayant une faible perméabilité et une faible solubilité. Dans le cas de ces principes actifs, l'obtention d'une biodisponibilité compatible avec l'effet thérapeutique nécessite de formuler le principe actif sous forme de solution ou dispersion solide dans un polymère (par un procédé organique ou par extrusion à chaud).

Cependant, il est difficile de stabiliser des solutions et dispersions solides ce qui conduit à une recristallisation qui peut provoquer une perte de biodisponibilité au cours du temps. Les solutions/dispersions solides obtenues par un procédé organique présentent l'inconvénient de contenir des traces de solvants organiques.

D'autre part, les procédés par extrusion à chaud ne sont pas utilisables pour des molécules thermolabiles et le choix des excipients envisageables est limité aux composés thermofusibles.

La fabrication de films fins incluant un principe actif en suspension est complexe. En effet, les particules non solubilisées tendent à sédimenter rapidement ce qui induit des problèmes d'homogénéité de teneur en principe actif ainsi que des défauts d'aspect sur le produit fini.

Une alternative à la fabrication de solutions solides pour augmenter la biodisponibilité des principes actifs peu solubles est de diminuer la taille de particules du principe actif pour augmenter sa vitesse de solubilisation (équation de Noyes-Whitney, l'équation classique développée relative à la vitesse de dissolution d'une substance dans un solvant) et ainsi maximiser son absorption in-vivo.

Une nanosuspension de principe actif peut être stabilisée par des agents de surface ce qui limite le phénomène de maturation d'Ostwald, phénomène qui se produit au cours du vieillissement des suspensions. Il s'agit d'une migration des molécules constituant la particule de plus petite taille, jusqu'à la particule de plus grande taille. Ce phénomène entraîne une augmentation de la taille moyenne des particules de phase dispersée, et un resserrement de la répartition granulométrique de la suspension. La nanosuspension garantit donc une plus grande homogénéité de la taille des particules au cours du temps. De plus, la nanosuspension peut être directement utilisée dans le procédé de fabrication du film par rajout des polymères filmogènes, ce qui facilite sa mise en oeuvre.

De manière inattendue la demanderesse a réussi à développer un procédé simple et efficace permettant de réduire la taille des particules d'un ou plusieurs principes actifs à un niveau nanométrique juste avant son/leur inclusion dans un film fin destiné à la voie orale et/ou buccale.

L'objet de la présente invention est de fournir une composition orale et/ou buccale sous forme de film fin ne présentant pas les inconvénients des films de l'état de la technique. Ce film est homogène (teneur en principe actif et aspect), stable dans le temps, notamment en ce qui concerne la taille des particules de principe actif et évite toute utilisation de solvants autres que de l'eau pour sa fabrication.

En particulier, un objet de l'invention est de fournir un film fin soluble permettant d'éviter l'inhomogénéité du principe actif au sein du film par l'utilisation d'une nanosuspension tout en augmentant la biodisponibilité du principe actif.

Le terme nanosuspension selon l'invention doit être compris comme étant une suspension dans un solvant dans lequel le principe actif n'est pas soluble, de préférence l'eau, du principe actif broyé très finement dont au moins 90% des particules (D(90)) ont une taille inférieure à 1000 nm, de préférence inférieure à 800 nm, et plus préférentiellement encore inférieure à 600 nm. La taille des particules est déterminée par granulométrie en diffraction laser ou en diffusion de la lumière.

Par biodisponibilité on entend la fraction de la dose de médicament administré qui atteint la circulation générale.

Ainsi, selon un premier objet, l'invention porte sur une composition orale et/ou buccale sous forme de film fin d'un principe pharmaceutiquement actif faiblement soluble dans l'eau et les fluides du tractus grastro-intestinal, comprenant des particules dudit principe actif dispersées dans un polymère filmogène, au moins 50% en poids du poids total de principe actif présentant une répartition granulométrique telle qu'au moins 90% desdites particules ont une taille inférieure à 1000 nm, de préférence inférieure à 800 nm, et plus préférentiellement encore inférieure à 600 nm.

Les polymères filmogènes utilisés pour la fabrication des compositions selon la présente invention, notamment des films orodispersibles, peuvent être d'origine naturelle tels que les pectines, les dérivés d'amidon tels les pullulanes ou les dérivés d'algues comme par exemple les alginates. Des dérivés cellulosiques peuvent aussi être utilisés tels que l'éthylcellulose, l'hydroxypropylméthylcellulose, hydroxypropylcellulose, hydroxyméthylcellulose, l'hydroxyéthylcellulose; ainsi que des celluloses microcristallines. Les polymères hydrophiles biodégradables peuvent également être utilisés dans les compositions de la présente invention, notamment les alcools polyvinyliques et alcools polyvinyliques modifiés ou encore les polyvinylpyrrolidones (FVP), aussi appelées polyvidone ou povidone, les polyols tels que les polyéthylène glycols (PEG) aussi appelés polyoxyéthylène, et les amidons modifiés.

Selon l'invention la base polymérique utilisée pour la fabrication du film peut être un seul polymère ou un mélange de différents polymères dans différents ratios.

Les principes actifs utilisés dans les compositions décrites dans la présente invention sont des principes actifs peu solubles dans l'eau et les fluides du tractus gastro-intestinal. Ils sont classés dans les classes II et IV de la classification BCS, sans toutefois s'y limiter.

De façon préférentielle, les principes actifs utilisés dans les compositions décrites dans la présente invention peuvent être sélectionnés parmi une variété de classes de médicaments connus, comme par exemple les analgésiques, les produits de substitution aux opiacées, les anti-inflammatoires, les anti-arythmiques, les antibiotiques (y compris les pénicillines), les anticoagulants, les antidépresseurs, les antiépileptiques, les antihistaminiques, les antihypertenseurs, les immunosuppresseurs, les sédatifs anxiolytiques (hypnotiques et neuroleptiques), les astringents, les bêta-bloquants adrénergiques, les agents suppresseurs de la toux, les agents régulation des lipides, des relaxants musculaires, des vasodilatateurs et des inhibiteurs de la phosphodiestérase, tels que des xanthines, mais ne sont pas limités à ces classes thérapeutiques. Les principes actifs préférés comprennent ceux destinés à être administrés par voie orale et/ou buccale.

Selon un mode de réalisation de l'invention, le principe actif subit un nanobroyage dans sa totalité. La composition sous forme de film fin comprend donc le principe actif entièrement nanonisé. Dans ce mode de réalisation, 90% des particules (D(90)) ont une taille inférieure à 1000 nm, de préférence inférieure à 800 nm et plus préférentiellement encore inférieure à 600 nm. La taille des particules est déterminée par granulométrie en diffraction laser ou en diffusion de la lumière.

Selon une autre variante de l'invention, seule une partie du principe actif a subi un nanobroyage. L'autre partie a uniquement été dispersée dans la nanosuspension. De façon préférentielle, la partie de principe actif nanobroyé est majoritaire (> 50% poids/poids).

Selon l'invention il peut aussi être envisagé que la composition comprenne un mélange de plusieurs principes actifs de solubilités différentes, le principe actif présentant la moins bonne solubilité est nano broyé au moins en partie, et le (ou les) autre(s) principe(s) actif(s) est(sont) dispersé(s) dans la nanosuspension.

De préférence, les principes actifs utilisés dans la composition selon l'invention sont des principes actifs pharmaceutiques peu solubles dans l'eau ou les fluides du tractus gastro-intestinal et sont inclus dans la composition en une quantité allant d'environ 0,01% à 80%, de préférence 1% à 50% (poids/poids de la composition).

Selon un mode de réalisation particulier, l'invention concerne une forme pharmaceutique orale sous forme de film fin unitaire appropriée pour l'administration orale comprenant environ 0,1 mg à environ 100 mg du principe actif par film fin unitaire.

Des additifs ou excipients peuvent également être ajoutés à la composition selon l'invention. Ceux-ci peuvent inclure, sans toutefois s'y limiter: des agents mouillants tels que des tensioactifs, des plastifiants de type polyalcools; des anti-moussants, comme les composés siliconés, qui favorisent une surface lisse du film en favorisant l'élimination des bulles d'air dans le film; des gélifiants tels que les gels de pectine, carraghénane, et la gélatine, ce qui aide à maintenir la dispersion des composants, des composés d'inclusion, tels que les cyclodextrines.

Parmi les additifs ou excipients on peut également inclure des lubrifiants, des stabilisants, des agents viscosifiants, des colorants, des édulcorants, des arômes, des accélérateurs de coulée, des diluants, des liants, des tampons pH, des agents de glissement.

Selon un mode de réalisation particulier, les compositions pharmaceutiques de la présente invention comprennent un ou plusieurs polymères et au moins un principe actif, et différents excipients choisis parmi un agent mouillant (tensioactif), un agent anti-mousse, et leurs mélanges, et optionnellement un agent viscosifiant, un arôme, un édulcorant et/ou un colorant.

Les agents anti-mousse pouvant être utilisés dans la présente invention sont des dérivés du silicone. Le siméthicone est particulièrement utile comme agent anti-mousse. L'utilisation d'un agent anti-mousse cependant n'est pas limitée à l'utilisation du siméthicone, ainsi d'autres agents anti-mousse peuvent être utilisés.

Les agents viscosifiants pouvant être utilisés dans la présente invention sont les dérivés de celluloses, les gélatines, des gommes naturelles ou modifiées, ou d'autres polymères permettant d'augmenter la viscosité de l'eau.

Avantageusement l'agent viscosifiant utilisé dans les compositions pharmaceutiques de l'invention est un dérivé de cellulose. De préférence, le dérivé cellulosique est l'hydroxypropylméthylcellulose.

On choisit de préférence l'hydroxypropyl-méthylcellulose (HPMC) ou hypromellose dont la viscosité apparente est de 3 à 15 mPa.s et de manière encore plus préférée de 3 à 6 mPa.s, comme par exemple le Pharmacoat 603® (HPMC de faible viscosité) et peut être ajouté au mélange de formation de film en une quantité d'environ 0,1 pour cent en poids à environ 50,0 pour cent en poids de la nanosuspension, plus avantageusement d'environ 1,0 pour cent en poids à environ 20,0 pour cent en poids de la nanosuspension, et plus avantageusement d'environ 1,0 pour cent en poids à environ 10,0 pour cent en poids de la nanosuspension.

Le tensioactif est choisi parmi les tensioactifs solides ou liquides à température ambiante, par exemple le laurylsulfate de sodium, le Polysorbate 80 ou le Montane 20, de préférence le laurylsulfate de sodium.

Avantageusement le principe actif est un inhibiteur de la phosphodiestérase de préférence le tadalafil compris dans une nanosuspension aqueuse. Cette suspension inclut aussi un dérivé cellulosique en tant qu'agent viscosifiant, un tensioactif en tant qu'agent mouillant et un anti-mousse. Le tadalafil est de préférence sous forme cristalline, la taille des particules (D(50)) étant inférieure à 1000 nm, de préférence inférieure à 800 nm, et plus préférentiellement encore inférieure à 600 nm.

Selon une variante, le tadalafil est sous forme cristalline, la taille des particules (D(90)) étant inférieure à 1000 nm, de préférence inférieure à 800 nm et plus préférentiellement encore inférieure à 600 nm.

La composition sous forme de film fin selon l'invention permet de mettre à disposition des formes de dosage unitaires dans lesquelles le principe actif est dispersé de façon homogène. Ces formes de dosage unitaires sont facilement prises par le patient ce qui permet d'améliorer l'observance des traitements. Elles se désintègrent très rapidement en bouche, notamment sur les muqueuses et facilitent ainsi une action rapide de l'actif.

La présente invention concerne également un procédé de préparation du film fin à usage oral et/ou sublingual. Selon le procédé général de l'invention, le principe actif est mis en suspension dans un solvant dans lequel il n'est pas soluble, puis nanonisé. La nanosuspension obtenue est additionnée de polymère filmogène, puis la composition obtenue est mise sous la forme de films fins.

Ainsi, suivant le procédé de préparation d'une composition selon l'invention, successivement:
- on met en suspension le principe actif dans un solvant dans lequel il n'est pas soluble,
- on broie les particules de principe actif,
- on ajoute au moins un polymère filmogène,
- on transforme la composition ainsi obtenue en films fins.

Selon un mode de réalisation particulier, ledit procédé comprend les étapes suivantes :
a/ mettre un principe actif en suspension dans un solvant dans lequel ledit principe actif n'est pas soluble;
b/ éventuellement, ajouter à la dispersion un agent mouillant avec éventuellement d'autres excipients;
c/ broyer le mélange obtenu à l'étape précédente dans un broyeur à billes jusqu'à l'obtention d'une granulométrie moyenne du principe actif (D (50)) inférieure à 1000 nm, de préférence inférieure à 800 nm et plus préférentiellement encore inférieure à 600 nm;
d/ mélanger de manière homogène la nanosuspension obtenue avec au moins un polymère et éventuellement des excipients;
e/ couler la composition obtenue à l'étape d) sur un support pour obtenir un film;
f/ sécher le film et éventuellement le découper.

Le principe actif est dans un premier temps mis en suspension dans un solvant dans lequel il est non soluble. Ce solvant peut être de toute nature, organique ou non. De préférence, le solvant utilisé pour la présente invention est l'eau. Certains excipients peuvent aussi être ajoutés à cette suspension.

D'une façon préférentielle mais non limitative, il est ajouté à cette suspension un agent mouillant permettant une bonne dispersion du principe actif. Cet agent mouillant peut être de nature variée, mais de façon préférentielle un tensio-actif est utilisé, par exemple du laurylsulfate de sodium ou ses dérivés. L'agent tensioactif représente entre 0,1 à 5%, de préférence entre 0,3 et 3% en poids de la nanosuspension, les % étant exprimés par rapport au poids total de la nanosupension.

Un agent anti-mousse peut également être ajouté pour éviter la formation importante de mousse. Cet agent est par exemple un dérivé de silicone, notamment la siméthicone, mais peut être de nature assez variée.

L'agent anti-mousse utilise dans les compositions pharmaceutiques peut être ajouté en une quantité d'environ 0,01 % en poids à environ 5,0 % en poids de la nanosuspension, plus avantageusement d'environ 0,02 % en poids à environ 1,0 % en poids de la nanosuspension, et plus avantageusement d'environ 0,05 % en poids à environ 0, 5 % en poids de la nanosuspension, les % étant exprimés par rapport au poids total de la nanosupension.

Un agent viscosifiant peut aussi être ajouté pour faciliter le maintien en suspension des particules de principes actifs. Tout polymère viscosifiant peut être utilisé à cette fin.

Il peut être ajouté en une quantité d'environ 0,1 % en poids à environ 50,0 % en poids de la nanosuspension, plus avantageusement d'environ 1,0 % en poids à environ 20,0 % en poids de la nanosuspension, et plus avantageusement d'environ 2,0 % en poids à environ 10,0 % en poids de la nanosuspension, les % étant exprimés par rapport au poids total de la nanosuspension.

La suspension obtenue est par la suite introduite dans un nanobroyeur à billes en voie humide. Cet équipement permet un broyage des particules de principe actif par effet mécanique. Des billes insolubles de taille définie s'entrechoquent et cassent les particules de principe actif jusqu'à une taille minimale. La taille des billes de broyage est inférieure à 1 mm et de façon préférentielle inférieure à 800 pm, de préférence inférieure à 600 pm.

De préférence, la suspension à broyer circule dans le nanobroyeur en circuit fermé de telle sorte que le broyage soit le plus efficace possible.

Le broyage est arrêté lorsque l'on obtient une granulométrie moyenne du principe actif (D (50)) inférieure à 1000 nm, de préférence 800 nm et plus préférentiellement 600 nm.

Une fois la nanosuspension fabriquée, on ajoute le polymère filmogène et éventuellement les autres excipients permettant la fabrication du film fin sont ajoutés sous agitation. On peut également ajouter un autre principe actif ou des particules non nano-broyées du principe actif déjà présent dans la nanosuspension. Une fois un mélange homogène obtenu, la suspension est coulée sur un support puis séchée dans un four. Le produit est par la suite détaché de son support et découpé à la taille désirée permettant de former des films unitaires, c'est-à-dire les unités de prises.

De manière préférentielle mais non limitative, le film selon l'invention présente une teneur finale en eau de 1 % à 5 % (m/m), de préférence inférieure à 4,5 % (m/m), et plus préférentiellement encore inférieure à 4 % (m/m). La faible teneur finale en eau favorise la stabilité du film au cours du temps.

La présente invention porte aussi sur l'utilisation d'une composition pour la fabrication d'un médicament pour l'administration jusqu'à une dose totale maximale de 20 mg de tadalafil par jour pour le traitement du dysfonctionnement sexuel chez un patient qui en a besoin.

L'invention n'est pas limitée aux seuls exemples de réalisations explicitement décrits ci-dessus.

### EXEMPLES :

Dans les exemples ci-dessous, les pourcentages sont des pourcentages en poids/poids.

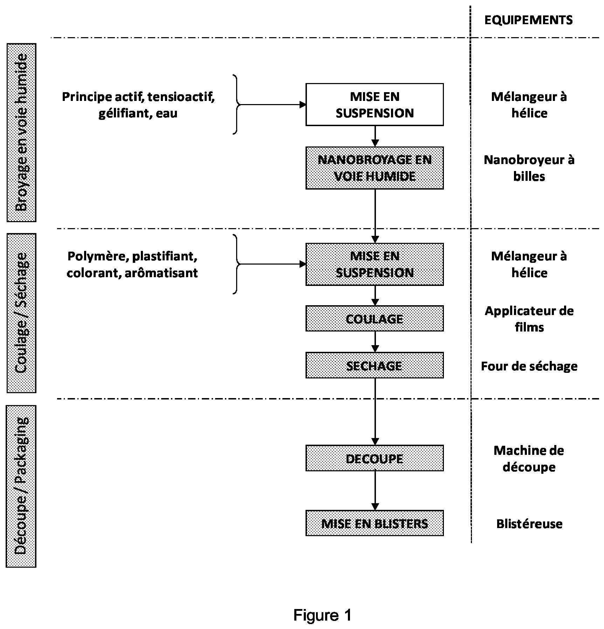

Le procédé mis en oeuvre dans les exemples ci-dessous est représenté de façon générale par le diagramme de la figure 1.

### Exemple 1. Préparation d'une suspension nanobroyée de tadalafil.

Une suspension aqueuse comprenant 500 mg de tadalafil est préparée de la façon suivante.

De l'HPMC 603 est mise en suspension dans de l'eau à température ambiante dans un contenant adapté et sous agitation à l'aide d'une hélice marine. Puis du laurylsulfate de sodium et une émulsion de siméthicone sont ajoutés, toujours sous agitation. Enfin, le principe actif est ajouté à ce mélange sous agitation jusqu'à l'obtention d'une suspension visuellement homogène.

Un agent tensioactif, le laurylsulfate de sodium est ajouté pour faciliter la mise en suspension du tadalafil.

Un agent anti-mousse, le siméthicone est ajouté à la suspension pour éviter toute formation de bulles.

Un agent viscosifiant, un dérivé cellulosique l'HPMC 603 est aussi introduit pour favoriser le maintien du principe actif en suspension et favoriser l'efficacité du broyage. La formule de la suspension de broyage est décrite au tableau 1.

**Tableau 1 : Formule finale des films.**

| | Suspension de Tadalafil | |
|---|---|---|
| composants | Quantité (g) | Composition centésimale* |
| Tadalafil | 500,0g | 20,0% |
| Laurylsulfate de sodium | 50,0g | 2,0% |
| Siméthicone | 5,0g | 0,2% |
| HPMC 603 | 62,5g | 2,5% |
| Eau purifiée | 1882,5g | 75,3% |
| Total | 2500,0g | 100,0% |

Cette suspension est préparée dans une cuve de taille adaptée (bécher de grande contenance en inox) et mélangée à l'aide d'un mélangeur à hélice (un mélangeur IKA à hélice marine) le temps nécessaire à l'obtention d'une suspension homogène, le temps total de mélange est d'environ une heure.

Cette suspension est ensuite nanobroyée dans un broyeur à billes en voie humide jusqu'à obtention d'une suspension dont la distribution granulométrique de la phase dispersée présente une taille maximale inférieure à 1000 nm (taille nanométrique).

### Exemple 2: Préparation des films orodispersibles de tadalafil.

La suspension obtenue à l'exemple 1 est ensuite utilisée comme base pour la fabrication de films orodispersibles.

On ajoute à ladite suspension le polymère qui sert à former le film, qui est du pullulane, le glycérol comme plastifiant et un arôme citron.

La formulation quantitative est décrite au tableau 2.

**Tableau 2 : Formule finale des films.**

| | | Quantité (g) | Composition centésimale* |
|---|---|---|---|
| Suspension nanonisée de tadalafil | Tadalafil | 500,0g | 19,42% |
| | Laurylsulfat e de sodium | 50,0g | 1,94% |
| | Siméthicone | 5,0g | 0,19% |
| | HPMC 603 | 62,5g | 2,43% |
| | Eau purifiée | 1882,5g | - |
| Pullulan | | 1340,0g | 52,05% |
| Glycérol | | 536,5g | 20,84% |
| Arôme citron | | 80,5g | 3,13% |
| Eau purifiée | | q.s. | - |
| Total masse sèche* | | 2679,5g | 100,00% |

La composition ainsi obtenue est coulée sur un support, le film ainsi formé est séché dans un four jusqu'à un taux de l'ordre de 5 à 10%.

### Exemple 3: Découpe des films orodispersibles de tadalafil.

Les films une fois séchés sont ensuite découpés à la taille désirée à l'emporte pièce. Classiquement la taille des films découpés varie de 1 à 2 mm de largeur pour 2 à 3 mm de longueur.

Ce procédé est appliqué au film formé à l'exemple 2. La formule finale centésimale d'un film unitaire de tadalafil nanonisé de 2 mm de largeur et 3 mm de longueur est décrite dans le tableau 3.

**Tableau 3 : film unitaire de tadalafil nanonisé de 2 mm de largeur et 3 mm de longueur.**

| | | Quantité (g) | Composition centésimale* |
|---|---|---|---|
| Suspension nanonisée de tadalafil | Tadalafil | 10,00 | 19,42% |
| | Laurylsulfat e de sodium | 1,00 | 1,94% |
| | Siméthicone | 0,10 | 0,19% |
| | HPMC 603 | 1,25 | 2,43% |
| Pullulane | | 26,80 | 52,05% |
| Glycérol | | 10,73 | 20,84% |
| Arôme citron | | 1,61 | 3,13% |
| Eau résiduelle | | qs. | - |
| Total masse sèche | | 42,87 | 100,0% |

### Exemple 4:Composition centésimale du film sec.

Les films présentés au tableau 4 ont été réalisés selon le même protocole que celui décrit dans les exemples 1, 2 et 3.

**Tableau 4 : Composition centésimale du film.**

| Composants | Masse par film sec (mg) | Quantité du film sec (%) |
|---|---|---|
| Tadalafil | 20.00 | 26.63 |
| Hypromellose 603 | 2.50 | 3.33 |
| Polysorbate 80 | 2.00 | 2.66 |
| Siméthicone (émulsion) | 0.20 | 0.27 |
| Hydroxypropylcellulose SSL | 46.32 | 61.68 |
| Glycérol | 3.76 | 5.01 |
| Sucralose | 0.32 | 0.42 |
| *Total (masse sèche)* | *75.10* | *100.00* |

Deux types de films sont issus de cette composition, un film dont le principe actif a été micronisé et le deuxième dont le principe actif a été nanonisé.

La teneur finale en eau de ces films a été mesurée par la méthode Karl Fisher (appareil Mettler Toledo).

La granulométrie D50 et D90 de ces films a été mesurée à l'aide d'un granulomètre (Mastersizer 2000) utilisant la diffraction laser pour mesurer la taille des particules du principe actif nanonisé et micronisé.

Les valeurs de granulométrie D50 et D90 et la teneur finale en eau de ces films répondant à la composition du tableau 4 sont présentées au tableau 5 ci-dessous.

**Tableau 5 : Granulométries des principes actifs utilisés et teneur finale en eau des deux films.**

| | D50 | D90 | Teneur finale en eau % (m/m) |
|---|---|---|---|
| Test 1 (tadalafil micronisé) | 4µm | 9µm | 5 % |
| Test 2 (tadalafil nanonisé) | 0,1µm | 0,2µm | 4,1 % |

### Données pharmacocinétiques des deux types de film (Test 1 : Tadalafil micronisé et Test 2 : Tadalafil macronisé)

**Tableau 6: données pharmacocinétiques des deux types de film (tadalafil micronisé et macronisé).**

| **Paramètres** | **TEST-1 (micronisé)** | | **TEST-2 (nanonisé)** | |
|---|---|---|---|---|
| | **Valeur** | **C.V. (coefficient de variation)** | **Valeur** | **C.V. (coefficient de variation)** |
| Cₘₐₓ | 375.15 | 28.9 | 454.94 | 23.8 |
| Tₘₐₓ (heures) | 3.00 | 33.8 | 2.50 | 26.4 |
| AUC_{0-T} | 7235.38 | 31.6 | 7810.18 | 32.5 |
| AUC_{0-∞} | 9444.30 | 44.2 | 10293.85 | 46.0 |

Au tableau 6 ci-dessus sont regroupées les valeurs des données pharmacocinétiques comparatives pour les deux films comprenant le tadalafil micronisé et nanonisé.

## Revendications

1. Composition orale et/ou buccale sous forme de film fin d'un principe pharmaceutiquement actif faiblement soluble dans l'eau et les fluides du tractus grastro-intestinal, comprenant des particules dudit principe actif dispersées dans un polymère filmogène, au moins 50% en poids du poids total de principe actif présentant une répartition granulométrique telle qu'au moins 90% desdites particules ont une taille inférieure à 1000 nm, de préférence inférieure à 800 nm et plus préférentiellement encore inférieure à 600 nm, ladite composition présentant une teneur finale en eau de 1 % à 5 % (m/m), de préférence inférieure à 4,5 % (m/m), et plus préférentiellement encore inférieure à 4 % (m/m).

2. Composition selon la revendication 1, dans laquelle la totalité des particules de principe pharmaceutiquement actif présente une répartition granulométrique telle qu'au moins 90% desdites particules ont une taille inférieure à 1000 nm, de préférence inférieure à 800 nm et plus préférentiellement encore inférieure à 600 nm.

3. Composition selon la revendication 1 ou 2, dans laquelle ledit principe actif appartient aux classes II et IV de la classification BCS.

4. Composition selon l'une quelconque des revendications 1 à 3, **caractérisée par le fait qu'**elle comprend en outre un excipient choisi parmi des agents mouillants, des plastifiants, des anti-moussants, des gélifiants, des composés d'inclusion, des lubrifiants, des stabilisants, des colorants, des édulcorants, des arômes, des accélérateurs de coulée, des diluants, des liants, des tampons pH, des agents de glissement, et leurs mélanges.

5. Composition selon l'une quelconque des revendications 1 à 4, **caractérisée par le fait que** le polymère est choisi dans le groupe comprenant les pectines, les amidons modifiés, les dérivés d'amidon tels que les pullulanes, les dérivés d'algues tels que les alginates, les celluloses microcristallines, les dérivés cellulosiques tels que l'éthylcellulose, l'hydroxypropylméthylcellulose, hydroxypropylcellulose, hydroxyméthylcellulose, l'hydroxyéthylcellulose, les polymères hydrophiles biodégradables, les alcools polyvinyliques, les alcools polyvinyliques modifiés, les polyvinylpyrrolidones (PVP), les polyols tels que les polyéthylène glycols (PEG), et leurs mélanges.

6. Composition selon l'une quelconque des revendications 1 à 5, qui est une forme pharmaceutique orale unitaire sous forme de film fin comprenant environ 0,1 mg à environ 100 mg du principe actif.

7. Composition selon l'une quelconque des revendications 1 à 6, **caractérisée par le fait qu'**elle comprend un autre principe actif.

8. Procédé de préparation d'une composition selon l'une quelconque des revendications 1 à 7, suivant lequel, successivement:
- on met en suspension le principe actif dans un solvant dans lequel il n'est pas soluble,
- on broie les particules de principe actif,
- on ajoute au moins un polymère filmogène,
- on transforme la composition ainsi obtenue en film fin.

9. Procédé de préparation d'une composition selon la revendication 8, comprenant les étapes suivantes :
a/ mettre un principe actif en suspension dans un solvant dans lequel il n'est pas soluble;
b/éventuellement, ajouter à la dispersion un agent mouillant avec éventuellement d'autres excipients;
c/ broyer le mélange obtenu à l'étape précédente dans un broyeur à billes jusqu'à l'obtention d'une granulométrie moyenne du principe actif (D (50)) inférieure à 1000 nm, de préférence inférieure à 800 nm et plus préférentiellement encore inférieure à 600 nm;
d/ mélanger de manière homogène la nanosuspension obtenue avec au moins un polymère et éventuellement des excipients;
e/couler la composition obtenue à l'étape d) sur un support pour obtenir un film;
f/ sécher le film et éventuellement le découper.

10. Procédé selon la revendication 8 ou 9, dans lequel ledit solvant est l'eau.

11. Composition selon l'une quelconque des revendications 1 à 7, contenant jusqu'à 20 mg de tadalafil pour son utilisation dans une méthode pour le traitement du dysfonctionnement sexuel.

## Patentansprüche

1. Orale und/oder bukkale Zusammensetzung in Form eines dünnen Films eines pharmazeutischen Wirkstoffs, der in Wasser und Flüssigkeiten des Magen-Darm-Traktes schlecht löslich ist, umfassend Teilchen des Wirkstoffs, die in einem filmbildenden Polymer dispergiert sind, wobei mindestens 50 Gew.-% des Gesamtgewichts des Wirkstoffs eine Teilchengrößenverteilung aufweist, bei der mindestens 90% der Teilchen eine Größe von weniger als 1000 nm, vorzugsweise weniger als 800 nm und noch bevorzugter weniger als 600 nm aufweisen, wobei die Zusammensetzung einen Wasserendgehalt von 1% bis 5% (m/m), vorzugsweise weniger als 4,5% (m/m) noch bevorzugter weniger als 4% (m/m) aufweist.

2. Zusammensetzung gemäß Anspruch 1, bei der alle Teilchen des pharmazeutischen Wirkstoffs eine Teilchengrößenverteilung aufweisen, bei der mindestens 90% der Teilchen eine Größe von weniger als 1000 nm, bevorzugt weniger als 800 nm und noch mehr bevorzugt weniger als 600 nm aufweisen.

3. Zusammensetzung gemäß Anspruch 1 oder 2, wobei der Wirkstoff zu den Klassen II und IV der BCS-Klassifizierung gehört.

4. Zusammensetzung gemäß einem der Ansprüche 1 bis 3, **dadurch gekennzeichnet, dass** sie ferner einen Hilfsstoff enthält, der ausgewählt ist aus Benetzungsmitteln, Weichmachern, Antischaummitteln, Geliermitteln, Einschlussverbindungen, Schmiermitteln, Stabilisatoren, Färbemitteln, Süßstoffen, Geschmacksstoffen, Gießbeschleunigern, Verdünnungsmitteln, Bindemitteln, pH-Puffern, Gleitmitteln und Mischungen davon.

5. Zusammensetzung gemäß einem der Ansprüche 1 bis 4, **dadurch gekennzeichnet, dass** das Polymer ausgewählt ist aus der Gruppe umfassend Pektine, modifizierte Stärken, Stärkederivate wie Pullulane, Algenderivate, wie Alginate, mikrokristalline Cellulosen, Cellulosederivate wie Ethylcellulose, Hydroxypropylmethylcellulose, Hydroxypropylcellulose, Hydroxymethylcellulose, Hydroxyethylcellulose, biologisch abbaubare hydrophile Polymere, Polyvinylalkohole, modifizierte Polyvinylalkohole, Polyvinylpyrrolidone (PVP), Polyole wie Polyethylenglycole (PEG) und Mischungen davon.

6. Zusammensetzung gemäß einem der Ansprüche 1 bis 5, welche in einer einheitlichen oralen pharmazeutischen Form in Form eines dünnen Films vorliegt, umfassend etwa 0,1 mg bis etwa 100 mg des Wirkstoffs.

7. Zusammensetzung gemäß einem der Ansprüche 1 bis 6, **dadurch gekennzeichnet, dass** sie einen weiteren Wirkstoff enthält.

8. Verfahren zur Herstellung einer Zusammensetzung gemäß einem der Ansprüche 1 bis 7, bei dem nacheinander
- der Wirkstoff in einem Lösungsmittel, in dem er nicht löslich ist, suspendiert wird,
- die Wirkstoffteilchen gemahlen werden,
- mindestens ein filmbildendes Polymer zugegeben wird,
- die so erhaltene Zusammensetzung zu einem dünnen Film verarbeitet wird.

9. Verfahren zur Herstellung einer Zusammensetzung gemäß Anspruch 8, umfassend die folgenden Schritte:
a/ Suspendieren eines Wirkstoffs in einem Lösungsmittel, in dem er nicht löslich ist;
b/ gegebenenfalls Zugeben eines Benetzungsmittels zu der Dispersion mit gegebenenfalls weiteren Hilfsstoffen;
c/ Mahlen der im vorherigen Schritt erhaltenen Mischung in einer Kugelmühle, bis dass eine mittlere Teilchengröße des Wirkstoffs (D(50)) von weniger als 1000 nm, vorzugsweise weniger als 800 nm und noch mehr bevorzugt weniger als 600 nm erhalten wird;
d/ gleichmäßiges Vermischen der erhaltenen Nanosuspension mit mindestens einem Polymer und gegebenenfalls Hilfsstoffen;
e/ Gießen der in Schritt d) erhaltenen Zusammensetzung auf einen Träger, um einen Film zu erhalten;
f/ Trocknen und gegebenenfalls Zuschneiden des Films.

10. Verfahren gemäß Anspruch 8 oder 9, wobei das Lösungsmittel Wasser ist.

11. Zusammensetzung gemäß einem der Ansprüche 1 bis 7, die bis zu 20 mg Tadalafil enthält, zur Verwendung in einem Verfahren zur Behandlung sexueller Funktionsstörung.

## Claims

1. An oral and/or buccal composition in the form of a thin film of a pharmaceutically active ingredient which has low solubility in water and gastrointestinal tract fluids, comprising particles of said active ingredient dispersed in a film-forming polymer, at least 50% by weight of the total weight of active ingredient having a particle size distribution such that at least 90% of said particles have a size of less than 1000 nm, preferably less than 800 nm and even more preferentially less than 600 nm, said composition having a final water content of from 1% to 5% (w/w), preferably less than 4.5% (w/w), and even more preferentially less than 4% (w/w).

2. The composition as claimed in claim 1, wherein all of the particles of pharmaceutically active ingredient have a particle size distribution such that at least 90% of said particles have a size of less than 1000 nm, preferably less than 800 nm and even more preferentially less than 600 nm.

3. The composition as claimed in claim 1 or 2, wherein said active ingredient belongs to classes II and IV of the BCS classification.

4. The composition as claimed in any one of claims 1 to 3, **characterized in that** it also comprises an excipient chosen from wetting agents, plasticizers, antifoams, gelling agents, inclusion compounds, lubricants, stabilizers, dyes, sweeteners, flavorings, flow accelerators, diluents, binders, pH buffers, slip agents, and mixtures thereof.

5. The composition as claimed in any one of claims 1 to 4, **characterized in that** the polymer is chosen from the group comprising pectins, modified starches, starch derivatives such as pullulans, algal derivatives such as alginates, microcrystalline celluloses, cellulose-based derivatives such as ethylcellulose, hydroxypropylmethylcellulose, hydroxypropylcellulose, hydroxymethylcellulose or hydroxyethylcellulose, biodegradable hydrophilic polymers, polyvinyl alcohols, modified polyvinyl alcohols, polyvinylpyrrolidones (PVPs), polyols such as polyethylene glycols (PEGs), and mixtures thereof.

6. The composition as claimed in any one of claims 1 to 5, which is in a unitary oral pharmaceutical form in the form of a thin film comprising approximately 0.1 mg to approximately 100 mg of the active ingredient.

7. The composition as claimed in any one of claims 1 to 6, **characterized in that** it comprises another active ingredient.

8. A process for preparing a composition as claimed in any one of claims 1 to 7, according to which, successively:
- the active ingredient is suspended in a solvent in which it is not soluble,
- the particles of active ingredient are milled,
- at least one film-forming polymer is added,
- the composition thus obtained is converted into a thin film.

9. The process for preparing a composition as claimed in claim 8, comprising the following steps:
a/ suspending an active ingredient in a solvent in which it is not soluble;
b/ optionally, adding, to the dispersion, a wetting agent with optionally other excipients;
c/ milling the mixture obtained in the previous step, in a ball mill until an average particle size of the active ingredient (D (50)) of less than 1000 nm, preferably less than 800 nm and even more preferentially less than 600 nm is obtained;
d/ uniformly mixing the nanosuspension obtained with at least one polymer and optionally excipients;
e/ casting the composition obtained in step d) onto a substrate so as to obtain a film;
f/ drying the film and optionally cutting it up.

10. The process as claimed in claim 8 or 9, wherein said solvent is water.

11. Composition as claimed in any one of claims 1 to 7, containing up to 20 mg of tadalafil for its use in a method for the treatment of sexual dysfunction.
